# EUROPEAN PATENT APPLICATION

(11) **EP 3 954 274 A1**
(43) Date of publication of application: **16.02.2022**
(21) Application number: 19856468.4
(22) Date of filing: 01.08.2019
(51) Int. Cl.: A61B 5/00

(54) **PHOTOPLETHYSMOGRAPHY APPARATUS AND ELECTRONIC DEVICE**

(30) Priority: 14.09.2018 CN 201811075505; 14.09.2018 CN 201821514031 U
(71) Applicant: Anhui Huami Information Technology Co., Ltd., Hefei, Anhui 230088 (CN)
(72) Inventor: LI, Kai, Hefei, Anhui 230088 (CN); HOU, Xianchang, Hefei, Anhui 230088 (CN)
(74) Representative: 2K Patentanwälte Blasberg Kewitz & Reichel
(86) International application number: PCT/CN2019/098825
(87) International publication number: WO 2020/052364

(57) **Abstract**

The present disclosure relates to a photoplethysmograph device and an electronic apparatus, and belongs to the technical field of terminals. The photoplethysmograph device includes a housing provided with a mounting hole; an optical device accommodated in the mounting hole; and a light-transmitting member including a central portion and a surrounding portion disposed around the central portion. The surrounding portion includes at least one end face having a textured layer configured to scatter and/or absorb light; the central portion has an end face without being provided with the textured layer. The light-transmitting member is connected with the housing and located at an outer side of the optical device. The end face of the central portion is disposed corresponding to the optical device, and the end face of the surrounding portion is disposed corresponding to an area without being provided with the optical device. The end face of the central portion near the optical device protrudes from the end face of the surrounding portion near the optical device.

## Description

### FIELD

The present disclosure relates to a technical field of terminals, and in particular, to a photoplethysmograph device and an electronic apparatus.

### BACKGROUND

A photoplethysmograph device is a detection device that acquires physiological signals by detecting photoplethysmographic signals of human bodies. Typically, the photoplethysmograph device includes a housing, and an optical device for acquiring photoplethysmographic signals. Moreover, the housing is provided with a mounting hole to accommodate the optical device.

In general, in order to obtain signals worth of analysis, it is necessary to ensure that the optical device can emit or receive light of sufficient intensity. Therefore, a size of the mounting hole is usually larger than a size of the optical device to avoid diminishing passage of light. In this situation, an area of the mounting hole without being provided with the optical device is exposed, so that the user can observe some solder joints through the mounting hole, or even an unsightly part except an effective light-emitting or photosensitive area of a surface of the optical device, such as metal encapsulation, electrode points, and associated wiring. The aesthetics of the photoplethysmograph device is degraded, and meanwhile the safety of the photoplethysmograph device is affected.

### SUMMARY

The present disclosure provides a photoplethysmograph device and an electronic apparatus to solve the defects in the related art.

A first aspect of the present disclosure provides a photoplethysmograph device. The photoplethysmograph device includes: a housing provided with a mounting hole; an optical device accommodated in the mounting hole; and a light-transmitting member including a central portion and a surrounding portion disposed around the central portion. The surrounding portion includes at least one end face having a textured layer, and the textured layer is configured to scatter and/or absorb light; the central portion has an end face without being provided with the textured layer. The light-transmitting member is connected with the housing, blocks the mounting hole, and is located at a side of the optical device near an outside of the mounting hole. The end face of the central portion is disposed corresponding to the optical device, and the end face of the surrounding portion is disposed corresponding to an area not occupied by the optical device.

Optionally, the end face of the central portion near the optical device protrudes from the end face of the surrounding portion near the optical device.

Optionally, the optical device includes a light emitter and a light sensor. The end face of the central portion is disposed corresponding to an emission source of the light emitter, or the end face of the central portion is disposed corresponding to a photosensitive face of the light sensor.

Optionally, the end face of the central portion includes a flat face or a curved face.

Optionally, the end face of the central portion near the optical device includes a condensing face protruding towards an inside of the mounting hole.

Optionally, the end face of the central portion corresponding to the optical device is sized in such a way that the intensity of light passing through the central portion is greater than or equal to a minimum effective detection light intensity of the optical device.

Optionally, an end face of the central portion away from the optical device and an end face of the surrounding portion away from the optical device are connected into a flat face or a curved face.

Optionally, an end face of the central portion away from the optical device and an end face of the surrounding portion away from the optical device are connected into a curved face protruding towards the outside of the mounting hole.

Optionally, the end face of the surrounding portion near the optical device has the textured layer.

Optionally, the surrounding portion further includes a surrounding portion body, and the textured layer is disposed on the surrounding portion body.

Optionally, the surrounding portion is of an integral structure.

Optionally, the photoplethysmograph device further includes a circuit board disposed in the mounting hole, connected with the optical device, and covered by the surrounding portion.

A second aspect of the present disclosure provides an electronic apparatus including the above photoplethysmograph device provided by the first aspect.

The photoplethysmograph device and the electronic apparatus proposed by the present disclosure at least have the following beneficial effects.

The light-transmitting member is connected to the housing and located in the mounting hole, so that the optical device and the area in the mounting hole not occupied by the optical device are no longer directly exposed, thereby ensuring the safety of the apparatus and improving the aesthetics of the device. The central portion of the light-transmitting member allows the light to directly exit or enter without being scattered, thereby avoiding energy loss caused by scattering, and ensuring normal use of the photoplethysmograph device. The surrounding portion of the light-transmitting member has the textured layer capable of scattering or absorbing light, so that the observer cannot obtain a clear image about the structure of the mounting hole from the outside of the photoplethysmograph device. Moreover, the central portion protrudes from the surrounding portion so that more light is emitted through the central portion. The textured layer of the surrounding portion is far away from the portion in the mounting hole that is desired to be shielded, so as to achieve a better shielding effect and further beautify the appearance of the apparatus.

It should be understood that the above general description and the following detailed description are intended to be exemplary and illustrative rather than restrictive.

### BRIEF DESCRIPTION OF THE DRAWINGS

The accompanying drawings, which are incorporated in and constitute a part of this specification, illustrate embodiments consistent with the present disclosure and, together with the description, serve to explain the principles of the present disclosure.
FIG. 1 is a schematic diagram of a photoplethysmograph device according to an exemplary embodiment.
FIG. 2 is a schematic diagram of a photoplethysmograph device according to another exemplary embodiment.
FIG. 3 is a schematic diagram of a light-transmitting member according to an exemplary embodiment.
FIG. 4 is a schematic diagram of an optical device according to an exemplary embodiment.
FIG. 5 is a schematic diagram of a surrounding portion of a light-transmitting member in a photoplethysmograph device according to an exemplary embodiment.
FIG. 6 is a partial top view of a surrounding portion of a light-transmitting member in a photoplethysmograph device according to another exemplary embodiment.
FIG. 7 is a schematic diagram of an optical path of a light emitter portion in a photoplethysmograph device according to an exemplary embodiment.
FIG. 8 is a schematic diagram of an optical path of a light sensor portion in a photoplethysmograph device according to an exemplary embodiment.

### Reference numerals:

1: housing,
11: mounting hole,
2: optical device,
21: optical device casing,
22: optical device working portion,
23: junction,
3: light-transmitting member,
31: central portion,
32: surrounding portion,
321: textured layer,
322: surrounding portion body,
4: circuit board,
a: first emergent light, b: second emergent light, c: third emergent light, d: stray light, e: first incident light, f: second incident light.

### DETAILED DESCRIPTION

Exemplary embodiments of the present disclosure will be described in detail and examples of the embodiments will be illustrated in the drawings. When the following description refers to the drawings, unless specified otherwise, the same numbers in different drawings represent the same or similar elements. The implementations described in the following exemplary embodiments do not represent all implementations consistent with the present disclosure, and instead they are merely examples of devices and methods consistent with aspects of the present disclosure as detailed in the appended claims.

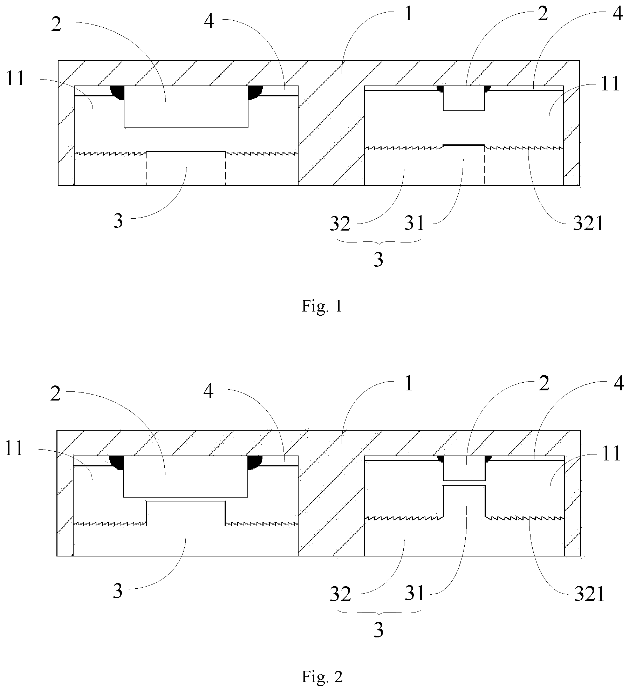

FIG. 1 is a schematic diagram of a photoplethysmograph device according to an embodiment of the present disclosure. A first aspect of embodiments of the present disclosure provides a photoplethysmograph device. As illustrated in FIG. 1, the photoplethysmograph device includes a housing 1, an optical device 2, and a light-transmitting member 3. The housing 1 is provided with a mounting hole 11, and the optical device 2 is accommodated in the mounting hole 11. The light-transmitting member 3 includes a central portion 31, and a surrounding portion 32 disposed around the central portion 31. Further, the surrounding portion 32 of the light-transmitting member 3 includes at least one end face having a textured layer 321, and the textured layer 321 is used to scatter and/or absorb light; the central portion 31 has an end face which is not provided with the textured layer 321.

The light-transmitting member 3 is connected to the housing 1, blocks the mounting hole 11 and is located at a side of the optical device 2 near an outside of the mounting hole 11. The end face of the central portion 31 is arranged corresponding to the optical device 2; the end face of the surrounding portion 32 is arranged corresponding to an area without being provided with the optical device 2.

The light-transmitting member 3 blocks the mounting hole 11 and is located at the side of the optical device 2 near the outside of the mounting hole 11, so that the light enters the light-transmitting member 3 through the end face of the light-transmitting member 3, and then enters or exits the mounting hole 11. The end face of the central portion 31 of the light-transmitting member 3 does not have the textured layer 321, so that light is hardly scattered and/or absorbed when passing through the central portion 31, thereby avoiding energy loss. The end face of the surrounding portion 32 has the textured layer 321 capable of scattering and/or absorbing light. When the textured layer 321 scatters the light, the textured layer 321 scatters the light emitted from the mounting hole 11 to disturb the light direction; when the textured layer 321 absorbs the light, part of the light emitted from the textured layer 321 is absorbed, weakening the light transmission of the surrounding portion 32. The light is scattered and/or absorbed by the textured layer 321, so that the observer cannot obtain a clear structural image about an inside of the mounting hole 11, thereby preventing the internal structure of the mounting hole 11 from being directly exposed, beautifying the appearance of the apparatus, and ensuring the safety of the apparatus.

Further, the light-transmitting member 3 has the central portion 31 and the surrounding portion 32, and it is possible to ensure that light of a relatively high intensity near an inside of light beam enters or exits without being scattered. Especially for an emergent light beam, the light of a smaller emergence angle in the light beam has higher energy and can be directly emitted through the central portion 31; the light of a larger emergence angle in the light beam has lower energy and can be emitted after being scattered and/or absorbed by the textured layer 321 of the surrounding portion 32. In such a case, the light emitted by the central portion 31 has relatively high energy, and a situation where light driving current needs to be increased due to the energy loss of the emergent light caused by the scattering and/or absorption of the textured layer 321 is avoided, thereby reducing the overall energy consumption of the photoplethysmograph device.

In the embodiment of the present disclosure, due to the light-transmitting member 3, the optical device 2 and the area in the mounting hole 11 without being provided with the optical device 2 are no longer directly exposed, thereby ensuring the safety of the apparatus and improving the aesthetics of the device. The central portion 31 of the light-transmitting member 3 allows the light of the sufficient intensity to enter or exit, to ensure the normal use of the photoplethysmograph device; the surrounding portion 32 shields the area in the mounting hole 11 without being provided with the optical device 2, thereby further beautifying the appearance of the apparatus and enhancing the structural security of the apparatus.

It should be noted that the end face of the central portion 31 near the optical device 2 protrudes from the end face of the surrounding portion 32 near the optical device 2, so that a side wall of a portion of the central portion 31 protruding from the surrounding portion 32 can totally reflect incident light of a large angle to allow more light as detection light to exit or enter the mounting hole 11, thereby improving the light utilization rate of the apparatus.

FIG. 2 is a schematic diagram of a light-transmitting member according to an exemplary embodiment. In one embodiment, as illustrated in FIG. 2, the end face of the central portion 31 near the optical device 2 protrudes from the end face of the surrounding portion 32 near the optical device 2.

The end face of the central portion 31 near the optical device 2 protrudes from the end face of the surrounding portion 32 near the optical device 2. The end face of the central portion 31 is closer to the optical device 2, so that more light is emitted through the central portion 31, thereby avoiding energy loss due to scattering. The end face of the surrounding portion 32 is remote from the optical device 2, such that the textured layer 321 is farther away from the portion in the mounting hole 11 that is desired to be shielded. It could be understood that the farther the textured layer 321 is from the portion that is desired to be shielded, the better the attainable shielding effect is.

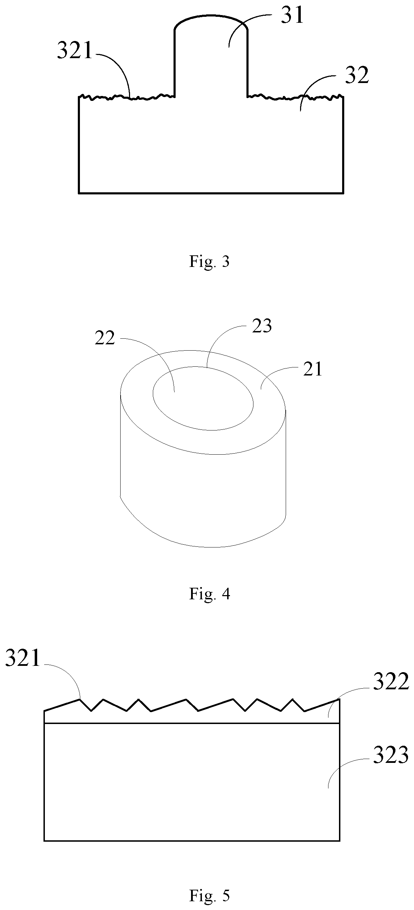

FIG. 3 is a schematic diagram of the light-transmitting member according to an exemplary embodiment. Regarding the configuration of the end face of the central portion 31, in one embodiment, optionally, the end face of the central portion 31 includes a flat face or a curved face, in which the curved face may be selected as a spherical or aspheric face. By means of the different configurations of the end face of the central portion 31, the light emission effect can be regulated to meet different use requirements. For example, as illustrated in FIG. 2, the end face of the central portion 31 near the optical device 2 includes a condensing face protruding towards the inside of the mounting hole 11. The aggregation degree of light passing through the central portion 31 can be enhanced by the condensing face. For the emergent light, the high aggregation degree helps to increase the probability of light entering a blood-rich skin layer; for the incident light, the high aggregation degree helps to generate stronger detection signals.

FIG. 4 is a schematic diagram of the optical device according to an exemplary embodiment. According to FIG. 4, the optical device 2 generally includes an optical device casing 21 and an optical device working portion 22. Exemplarily, when the optical device 2 is a light emitter, the optical device working portion 22 is an emission source; when the optical device 2 is a light sensor, the optical device working portion 22 is a photosensitive face.

In one embodiment, as illustrated in FIG. 1, and in conjunction with FIG. 4, the photoplethysmograph device includes two optical devices 2, namely a light emitter and a light sensor. The end face of the central portion 31 of the light-transmitting member 3 is disposed corresponding to an emission source of the light emitter, or the end face of the central portion 31 is disposed corresponding to a photosensitive face of the light sensor. That is, the end face of the central portion 31 of the light-transmitting member 3 is disposed corresponding to the optical device working portion 22. In such a case, a junction 23 of the optical device casing 21 and the optical device working portion 22 is shielded by the surrounding portion 32 of the light-transmitting member 3. It could be understood that the junction 23 of the optical device casing 21 and the optical device working portion 22 includes solder joints, a metal encapsulation edges, associated wiring and other structures. In this embodiment, by means of the surrounding portion 32, the observer is also unable to see the junction 23 clearly, thereby further beautifying the appearance of the photoplethysmograph device.

The light emitter can be a light emitting diode, a laser diode, a fluorescent lamp, an incandescent lamp, or an array of one or more light sources, and the like. Moreover, when the photoplethysmograph device has both the light emitter and the light sensor, the housing 1 is provided with mounting holes 11 for accommodating the light emitter 21 and the light sensor 22, respectively.

In one embodiment, the end face of the central portion 31 corresponding to the optical device 2 is sized in such a way that the intensity of the light passing through the central portion 31 is greater than or equal to the minimum effective detection light intensity of the optical device 2. The minimum effective detection light intensity refers to the minimum effective detection light intensity of the light sensor, and the light sensor can acquire a detection signal when the minimum effective detection light intensity is satisfied. In particular, as long as the minimum effective detection intensity is satisfied, the size of the end face of the central portion 31 can be smaller than or equal to the size of the emission source or the photosensitive face, so as to further prevent the observer from observing the structures in the mounting hole 11 and at the junction 23 from the outside. In such a case, the detection accuracy of the photoplethysmograph device and the aesthetics and safety of the apparatus can be simultaneously taken into account.

In one embodiment, an end face of the central portion 31 away from the optical device 2 and an end face of the surrounding portion 32 away from the optical device 2 are connected into a flat face or a curved face. Optionally, the curved face is a spherical or aspheric face, or a plurality of curved faces connected with and smoothly transitioning to each another. In such a case, the end face of the light-transmitting member 3 away from the optical device 2 has a smooth transition face, and has no sharp portion so as to avoid degrading tactile sensation.

Further, optionally, the end face of the central portion 31 away from the optical device 2 and the end face of the surrounding portion 32 away from the optical device 2 are connected to form a curved face, and the curved face protrudes towards the outside of the mounting hole 11. In use, the curved face of the light-transmitting member 3 protruding towards the outside of the mounting hole 11 can be pressed against the skin of the user to ensure that the emergent light enters the skin or the light reflected by the skin enters the light-transmitting member 3. Moreover, the curved face can be selected to have a large curvature, and hence will not cause an obvious oppressive feeling when used.

Further, in one embodiment, the end face of the surrounding portion 32 near the optical device 2 has the textured layer 321. The textured layer 321 is disposed at the end face of the surrounding portion 32 near the optical device 2, so that the end face of the light-transmitting member 3 away from the optical device 2 can be flat, further providing a good tactile sensation and optimizing the user experience.

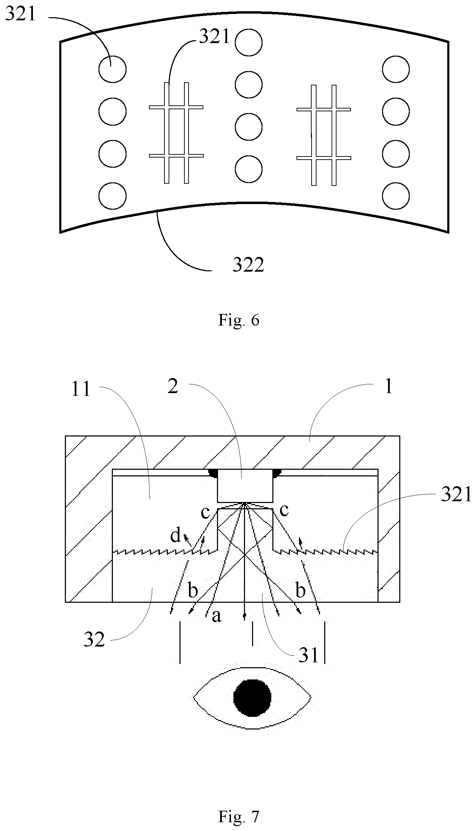

FIG. 5 is a schematic diagram of the surrounding portion of the light-transmitting member in the photoplethysmograph device according to an exemplary embodiment; FIG. 6 a partial top view of the surrounding portion of the light-transmitting member in the photoplethysmograph device according to another exemplary embodiment.

In one embodiment, as illustrated in FIG. 5, the surrounding portion 32 further includes a surrounding portion body 322, and the textured layer 321 is disposed on the surrounding portion body 322. In this embodiment, the surrounding portion 32 has a multi-layered structure, and when physical damages of the textured layer 321 diminish the shielding effect, it is easy to replace and repair, which facilitates the operation and maintenance. Exemplarily, the textured layer 321 is attached to the surrounding portion body 321.

Optionally, as illustrated in FIG. 5, the textured layer 321 is a textured film layer, and the textured layer 321 covers the end face of the surrounding portion 32. For example, the textured layer 322 may be an image shift film, a brightness enhancement film, or a frosted film.

Optionally, as illustrated in FIG. 6, the textured layer 321 includes a plurality of patches, and texture patterns are formed on the surrounding portion body 322 by the plurality of patches, to achieve scattering. The patch may be of a circular shape, a mesh shape, or the like, which is not specifically limited. Also, in this embodiment, the textured layer 321 is prepared using a material of low light transmittance, to absorb part of the light, thereby achieving the shielding effect.

In one embodiment, the surrounding portion 32 is of an integral structure, and the textured layer 321 can be processed by polishing, etching, mold forming, and the like.

It should be noted that, optionally, the textured layer 321 includes a regular pattern texture, such as a Fresnel lens texture formed by concentric circles, or includes an irregular pattern texture, such as a frosted texture. When the textured layer 321 includes a regular pattern texture, the texture has deep and pointed structural features to sufficiently scatter light; when the textured layer 321 includes an irregular pattern texture, the textured layer 321 has sufficient roughness to sufficiently scatter light.

In one embodiment, still with reference to FIG. 1, the photoplethysmograph device further includes a circuit board 4. The circuit board 4 is disposed in the mounting hole 11 and connected to the optical device 2, and the circuit board 4 is covered by the surrounding portion 32. By the scattering action of the textured layer 321 on the surrounding portion 32, the observer cannot clearly observe the wiring and other structures of the circuit board 4, and solder joints of the circuit board 4 and the optical device 2, from the outside of the photoplethysmograph device. In this case, the circuit board 4 is enclosed in the mounting hole 11 by the light-transmitting member 3 to prevent the adhesion of impurities from affecting the normal operation; moreover, by shielding the circuit board 4 with the surrounding portion 32, the circuit board 4 will not be exposed so as to avoid affecting the appearance of the apparatus, and the structure of the circuit board 4 becomes confidential.

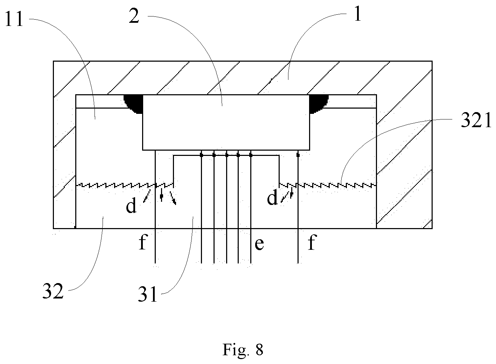

Based on the above, specific manners that the light enters and exits the photoplethysmograph device in the embodiments of the present disclosure will be described with reference to the accompanying drawings. FIG. 7 is a schematic diagram of an optical path of a light emitter portion in a photoplethysmograph device according to an exemplary embodiment; FIG. 8 is a schematic diagram of an optical path of a light sensor portion in a photoplethysmograph device according to an exemplary embodiment.

As illustrated in FIG. 7, the optical device 2 is a light emitter, and the emergent light emitted by the light emitter includes: first emergent light a directly emitted through the central portion 31; second emergent light b emitted after being totally reflected by a side wall of the central portion 31; third emergent light c emitted after being scattered by the textured layer 321, stray light d appearing during the scattering process.

The light emitting angles of the first emergent light a, the second emergent light b, and the third emergent light c are sequentially increased, and the energy thereof is sequentially decreased. The first emergent light a and the second emergent light b are not scattered by the textured layer 321 when emitted, and the energy loss is small. The third emergent light c is scattered by the textured layer 321 when emitted, and the energy loss is great. It is understood that the first emergent light a and the second emergent light b both having small light emitting angles and high energy can be directly emitted from the central portion 31 without being scattered, so as to ensure that the emitted light meets the minimum effective detection light intensity. Under the premise that the energy of the first emergent light a and the second emergent light b can satisfy the minimum effective detection light intensity of the light sensor, the third emergent light c with lower energy does not affect the detection effect of the photoplethysmograph device even if it is scattered.

As illustrated in FIG. 8, the optical device 2 is a light receiver light sensor, and the light receiver light sensor receives at least an optical signal generated by a first incident light e entering through the central portion 31. The first incident light e is not scattered, and the generated optical signal is strong, which helps the photoplethysmograph device to perform analysis according to the optical signal. It should be noted that when the photosensitive face is relatively large and the central portion 31 is relatively small, the photosensitive face also receives a second incident light f that is incident through the surrounding portion 32 and is scattered by the textured layer 321.

In addition, regarding the connection manner of the light-transmitting member 3 and the housing 1, optionally, the light-transmitting member 3 is connected to an inner wall of the mounting hole 11, or the light-transmitting member 3 is connected to a portion of the housing 1 outside the mounting hole 11 by means of a connecting member, which is not specifically limited in the embodiments of the present disclosure.

The photoplethysmograph device provided by the embodiments of the present disclosure can prevent the wiring and solder joints of the circuit board 4 in the mounting hole from being exposed, so as to ensure the safety of the photoplethysmograph device, beautify the appearance thereof, increase the luminous flux exiting or entering through the light-transmitting member 3, and improve the detection effect of the photoplethysmograph device.

A second aspect of embodiments of the present disclosure provides an electronic apparatus. The electronic apparatus includes the photoplethysmograph device provided by the above first aspect. The electronic device may be a smart wearable device, such as a bracelet or a foot ring, a medical detection device, or the like, which is not specifically limited in the embodiments of the present disclosure.

Other embodiments of the present disclosure will be apparent to those skilled in the art from consideration of the specification and practice of the present disclosure disclosed here. The present disclosure is intended to cover any variations, uses, or adaptations of the present disclosure following the general principles of the present disclosure and include common knowledge or conventional technical means in the art which are not disclosed in the present disclosure. The specification and embodiments should be regarded as illustrative only, and the true scope and spirit of the present disclosure is indicated by the following claims.

## Claims

1. A photoplethysmograph device, comprising:
a housing provided with a mounting hole;
an optical device accommodated in the mounting hole; and
a light-transmitting member comprising a central portion and a surrounding portion disposed around the central portion;
wherein the surrounding portion comprises at least one end face having a textured layer, and the textured layer is configured to scatter and/or absorb light; the central portion has an end face without being provided with the textured layer;
the light-transmitting member is connected with the housing, blocks the mounting hole, and is located at a side of the optical device near an outside of the mounting hole;
the end face of the central portion is disposed corresponding to the optical device, and the end face of the surrounding portion is disposed corresponding to an area without being provided with the optical device.

2. The photoplethysmograph device according to claim 1, wherein the end face of the central portion near the optical device protrudes from the end face of the surrounding portion near the optical device.

3. The photoplethysmograph device according to claim 1, wherein the optical device comprises a light emitter and a light sensor; the end face of the central portion is disposed corresponding to an emission source of the light emitter, or the end face of the central portion is disposed corresponding to a photosensitive face of the light sensor.

4. The photoplethysmograph device according to claim 1, wherein the end face of the central portion comprises a flat face or a curved face.

5. The photoplethysmograph device according to claim 1, wherein the end face of the central portion near the optical device comprises a condensing face protruding towards an inside of the mounting hole.

6. The photoplethysmograph device according to claim 1 or 3, wherein the end face of the central portion corresponding to the optical device is sized in such a way that an intensity of light passing through the central portion is greater than or equal to a minimum effective detection light intensity of the optical device.

7. The photoplethysmograph device according to claim 1, wherein an end face of the central portion away from the optical device and an end face of the surrounding portion away from the optical device are connected into a flat face or a curved face.

8. The photoplethysmograph device according to claim 1 or 7, wherein an end face of the central portion away from the optical device and an end face of the surrounding portion away from the optical device are connected into a curved face protruding towards the outside of the mounting hole.

9. The photoplethysmograph device according to claim 1, wherein the end face of the surrounding portion near the optical device has the textured layer.

10. The photoplethysmograph device according to claim 1, wherein the surrounding portion further comprises a surrounding portion body, and the textured layer is disposed on the surrounding portion body.

11. The photoplethysmograph device according to claim 1 or 10, wherein the surrounding portion is of an integral structure.

12. The photoplethysmograph device according to any one of claims 1 to 11, further comprising a circuit board disposed in the mounting hole, connected with the optical device, and covered by the surrounding portion.

13. An electronic apparatus, comprising a photoplethysmograph device according to any one of claims 1 to 12.
